Europäisches Patentamt

European Patent Office

Office européen des brevets

(11) Veröffentlichungsnummer: **0 166 332**

**B1**

## (12) EUROPÄISCHE PATENTSCHRIFT

(45) Veröffentlichungstag der Patentschrift:
**12.10.88**

(21) Anmeldenummer: **85107409.6**

(22) Anmeldetag: **15.06.85**

(51) Int. Cl.⁴: **G 01 N 11/14**

(54) **Rotationsviskosimeter.**

(30) Priorität: **27.06.84 DE 3423579**

(43) Veröffentlichungstag der Anmeldung:
**02.01.86 Patentblatt 86/1**

(45) Bekanntmachung des Hinweises auf die Patenterteilung:
**12.10.88 Patentblatt 88/41**

(84) Benannte Vertragsstaaten:
**DE FR GB IT**

(56) Entgegenhaltungen:
**DE-A-2 356 350**
**DE-A-2 742 229**
**FR-A-2 252 784**
**US-A-3 803 903**

(73) Patentinhaber: **Nukem GmbH, Rodenbacher Chaussee 6 Postfach 11 00 80, D-6450 Hanau 11 (DE)**

(72) Erfinder: **Weber, Manfred, Prof. Dr., Kaiserallee 15 b, D-7500 Karlsruhe (DE)**
Erfinder: **Dedegil, Mehmet Y. Dr., Berckmüllerstrasse 5, D-7500 Karlsruhe (DE)**

(74) Vertreter: **Nowak, Gerhard, DEGUSSA AG Fachbereich Patente Rodenbacher Chaussee Postfach 1345, D-6450 Hanau 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Rotationsviskosimeter zur Ermittlung der Fließeigenschaften von fließfähigen Gemischen mit grobkörnigen Komponenten, insbesondere von Betonmischungen, bestehend aus einem Antrieb mit Drehmoment-Meßeinrichtung, aus einem Topf und einem darin zentrisch gelagerten drehbaren Innenzylinder.

Die Förderung von feinkörnigen Suspensionen mit grobkörnigem Anteil, z. B. die Förderung von Fließbeton durch Rohre, gewinnt zunehmend an Bedeutung. Dabei ist die Kontrolle der Eigenschaften derartiger fließfähiger Gemische erforderlich. Kapillarviskosimeter sind aus Gründen der Kapillargeometrie sowie des Meßaufwandes bei kurz nacheinander erfolgenden Messungen über einen längeren Zeitraum nicht geeignet. Auch bekannte Rotationsviskosimeter, bestehend aus einem Topf und einem darin zentrisch gelagerten Innenzylinder, sind nicht geeignet, die Fließeigenschaften von Gemischen mit grobkörnigen Anteilen quantitativ zu bestimmen, da das zu prüfende Gemisch im Spalt unterhalb des Innenzylinders einen unerwünschten Beitrag zum gemessenen Drehmoment liefert, der das Ergebnis der definierbaren Scherströmung im Meßspalt beeinträchtigt.

Der Erfindung lag daher die Aufgabe zu Grunde, ein Rotationsviskosimeten zur Ermittlung der Fließeigenschaften von fließfähigen Gemischen mit grobkörnigen Komponenten, insbesondere von Betonmischungen, zu schaffen, bestehend aus einem Antrieb mit Drehmoment-Meßeinrichtung, aus einem Topf und einem darin zentrisch gelagerten drehbaren Innenzylinder, das eine einwandfreie Messung auch hoher Viskositäten gestattet, robust ausgelegt ist und eine Vielzahl von Messungen über einen längeren Zeitraum auf einfache Art gestattet.

Die Aufgabe wurde erfindungsgemäß dadurch gelöst, daß der Ringspalt zwischen dem Innenzylinder und dem Topf mindestens fünfmal größer ist als der größte Korndurchmesser der Feststoffe des in dem Ringspalt sich befindlichen Gemisches, daß der Innenzylinder mindestens bis zum unteren Rand sich in einer Sperrflüssigkeit befindet, die eine gegenüber der schwersten Komponente des Gemisches mindestens 1,5 mal so hohe Dichte aufweist, mit dem Gemisch nicht mischbar ist und mit diesem nicht reagiert, und daß die Oberfläche des Innenzylinders und/oder die Innenfläche des Topfes oberhalb des Sperrflüssigkeitsspiegels aufgerauht oder mit vertikalen Nuten versehen ist.

Es ist vorteilhaft, wenn die Sperrflüssigkeit aus Quecksilber besteht. Quecksilber ist wegen seiner hohen Dichte, seinem physikalischen Eigenschaften und seines chemischen Verhaltens gegenüber den meisten in Frage kommenden Meßsubstanzen als Sperrflüssigkeit besonders geeignet. Günstig ist es hinsichtlich des notwendigen Schlupfes der Feststoff-Flüssigkeitssuspensionen an der Wand, insbesondere an der Oberfläche des Innenzylinders, wo die maximale Schubspannung herrscht, wenn die Tiefe der Nuten die Hälfte der Breite der Nuten beträgt. Ebenso ist es vorteilhaft, wenn die Nuten achsensymmetrisch, also quer zur Strömungsrichtung, in verschiedenen Breiten angeordnet sind und die maximale Breite der Nuten dem maximalen Korndurchmesser der Feststoffkörner - je nach Probenzusammensetzung - entspricht.

Die Oberfläche des Innenzylinders und/oder die Innenfläche des Topfes dürfen im Bereich der Sperrflüssigkeit keine Nuten enthalten, damit Oberflächenwellen vermieden werden und das Kalibriermoment klein gehalten wird.

In der schematischen Abbildung ist das erfindungsgemäße Viskosimeter beispielhaft dargestellt.

Das Rotationsviskosimeter besteht aus einem Topf (1) und einem Innenzylinder (2). Der Innenzylinder (2) ist am Boden des Topfes (1), beispielsweise mit einer Zentrierkugel (12), gelagert, um die Radialschwingungen, die durch die grobdisperse Struktur der Meßsubstanz (6) hervorgerufen werden, zu vermeiden. Die Meßsubstanz (6) befindet sich ausschließlich im Ringspalt (3), wobei die Breite des Ringspaltes (3) mindestens fünfmal größer ist als der größte Korndurchmesser der Feststoffe in der Meßsubstanz (6). Zwischen dem Innenzylinder (2) und dem Boden des Topfes (1) befindet sich eine Sperrflüssigkeit (5), die eine gegenüber der schwersten Komponente des Gemisches (6) mindestens 1,5 mal so hohe Dichte aufweist, mit dem Gemisch (6) nicht mischbar ist und mit diesem nicht reagiert. Die Auswahl der Sperrflüssigkeit (5) ist abhängig von den Eigenschaften des Gemisches, d.h. der Meßsubstanz (6). Neben Quecksilber kann beispielsweise auch Tetrabromäthan als Sperrflüssigkeit (5) dienen.

Eine geringe Eintauchtiefe des Innenzylinders (2) in die Sperrflüssigkeit (5) reicht schon zur Kompensation des Gewichts des Innenzylinders (2) durch den Archimedischen Auftrieb aus. Dadurch wird erreicht, daß die Lagerreibung praktisch verschwindet. Außerdem wird durch die Sperrflüssigkeit (5) ausgeschlossen, daß die Meßsubstanz (6) unter den Innenzylinder (2) gelangt und die Lagerreibung verändert.

Die Wand des Innenzylinders (2) ist mit Nuten (7) versehen, die die Bodenkante (4) des Innenzylinders (2) nicht ganz erreichen und deren Enden in etwa bündig mit dem Sperrflüssigkeitsspiegel abschließen.

Die Handhabung des erfindungsgemäßen Rotationsviskosimeters erfolgt auf nachstehende Weise:

Der Innenzylinder (2), der mit einem Drehmomenten-Meßgerät und einem Antriebsmotor (11) gekoppelt ist, wird mittels einer Kurbel (9) am Stativ (8) abgelassen, bis er auf der Lagerkugel (12) am Topfboden sitzt. Nach Kontrolle der leichten Drehbarkeit wird die Kurbel (9) am Stativ (8) fixiert. Ein Justierring (10) dient der Justierung. Über den Ringspalt (3) wird Quecksilber als Sperrflüssigkeit (5) bis zur Unterkante der Nuten (7) eingefüllt. Die Füllhöhe beträgt minimal etwa das 2- bis 3-fache des größten Korndurchmessers.

Bei einer niedrigen Drehzahl wird die Meßsubstanz (6) in den Ringspalt (3) eingegeben. Die eingefüllte Masse der Meßsubstanz (6) wird festgehalten. Das Einfüllen bei drehendem Zylinder (2) sorgt für spontane Gleichverteilung der Meßsubstanz (6) im Spalt (3). Nach Beendigung des Einfüllens wird der Innenzylinder (2) gestoppt und die Messung wird programmgesteuert von n = 0 bis zur maximalen Drehzahl und wieder zurück auf n = 0 gefahren.

Nach Beendigung der Messung wird der Innenzylinder (2) durch Lösen der Kurbel (9) aus dem Meßtopf herausgefahren. Durch eine geeignete Formung des Topfbodens läßt sich die Trennung von Quecksilber und Meßsubstanz dadurch erreichen, daß das Quecksilber über einen Ablaßstutzen (13) abgelassen wird und nach Entfernen der Meßsubstanz aus dem Topf (1) wieder eingefüllt wird.

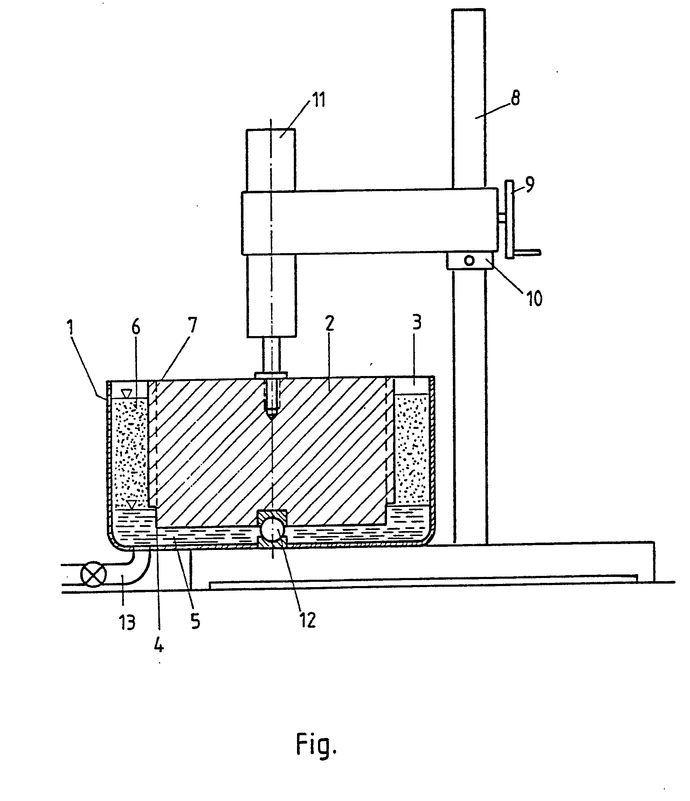

## Patentansprüche

1. Rotationsviskosimeter zur Ermittlung der Fliesseigenschaften von fließfähigen Gemischen mit grobkörnigen Komponenten, insbesondere von Betonmischungen, bestehend aus einem Antrieb mit Drehmoment-Meßeinrichtung, aus einem Topf und einem darin zentrisch gelagerten, drehbaren Innenzylinder,
dadurch gekennzeichnet,
daß der Ringspalt (3) zwischen dem Innenzylinder (2) und dem Topf (1) mindestens fünfmal größer ist als der größte Korndurchmesser der Feststoffe des in dem Ringspalt (3) sich befindlichen Gemisches (6), daß der Innenzylinder (2) mindestens bis zum unteren Rand (4) sich in einer Sperrflüssigkeit (5) befindet, die eine gegenüber der schwersten Komponente des Gemisches (6) mindestens 1,5 mal so hohe Dichte aufweist, mit dem Gemisch (6) nicht mischbar ist und mit diesem nicht reagiert, und daß die Oberfläche des Innenzylinders (2) und/oder die Innenfläche des Topfes (1) oberhalb des Sperrflüssigkeitsspiegels (5) aufgerauht oder mit vertikalen Nuten (7) versehen ist.

2. Rotationsviskosimeter nach Anspruch 1,
dadurch gekennzeichnet,
daß die Sperrflüssigkeit (5) aus Quecksilber besteht.

3. Rotationsviskosimeter nach Anspruch 1 und 2,
dadurch gekennzeichnet,
daß die Tiefe der Nuten (7) die Hälfte der Breite der Nuten (7) beträgt.

4. Rotationsviskosimeter nach Ansprüchen 1 bis 3,
dadurch gekennzeichnet,
daß die Nuten (7) achsensymmetrisch in verschiedenen Breiten angeordnet sind und die maximale Breite der Nuten (7) dem maximalen Korndurchmesser der Feststoffkörner entspricht.

## Claims

1. A rotary viscometer for determining the flow properties of flowable mixtures containing coarse-particled components, in particular of concrete mixtures, consisting of a drive means with torque measuring device, a pot and a rotatable inner cylinder arranged centrally therein, characterised in that the annular gap (3) between the inner cylinder (2) and the pot (1) is at least five times greater than the greatest particle diameter of the solid materials of the mixture (6) located in the annular gap (3), in that the inner cylinder (2) is located, at least to the lower edge (4), in a sealing liquid (5) which has a density at least 1.5 times as high as the heaviest component of the mixture (6), is immiscible with the mixture (6) and does not react with it, and in that the surface of the inner cylinder (2) and/or the internal surface of the pot (1) is roughened or is provided with vertical grooves (7) above the sealing liquid level (5).

2. A rotary viscometer according to claim 1, characterised in that the sealing liquid (5) is composed of mercury.

3. A rotary viscometer according to claim 1 and 2, characterised in that the depth of the grooves (7) is half the width of the grooves (7).

4. A rotary viscometer according to claims 1 to 3, characterised in that the grooves (7) are arranged axially symmetrically in various widths and the maximum width of the grooves (7) corresponds to the maximum particle diameter of the particles of solid materials.

## Revendications

1. Viscomètre rotatif pour la détermination des propriétés d'écoulement de mélanges fluides à composants à gros grains, en particulier de mélanges de béton, constitué d'un système d'entraînement avec dispositif de mesure du moment de torsion, d'un vase et d'un cylindre interne tournant, placé au centre dans celui-ci, caractérisé en ce que l'espace annulaire (3) entre le cylindre interne (2) et le vase (1) est au moins cinq fois plus grand que le plus grand diamètre des grains des matières solides du mélange (6) se trouvant dans l'espace annulaire (3), en ce que le cylindre interne (2) se trouve, au moins jusqu'au bord inférieur (4), dans un fluide d'arrêt (5) qui présente une densité égale au moins à 1,5 fois celle du composant le plus lourd du mélange (6), n'est pas miscible au mélange (6) et ne réagit pas avec celui-ci, et en ce que la surface du cylindre interne (2) et/ou la face interne du vase (1) est (sont) rendue(s) rugueuse(s) au-dessus du niveau du fluide d'arrêt (5) ou est (sont) pourvue(s) de rainures (7).

2. Viscomètre rotatif selon la revendication 1, caractérisé en ce que le fluide d'arrêt (5) est constitué de mercure.

3. Viscomètre selon les revendications 1 et 2, caractérisé en ce que la profondeur des rainures (7) est égale à la moitié de la largeur des rainures (7).

4. Viscomètre rotatif selon l'une quelconque des revendications 1 à 3, caractérisé en ce que les rainures (7) sont disposées en diverses largeurs symétriquement par rapport à l'axe, et la largeur maximale des rainures (7) correspond au diamètre maximum des grains de la matière solide.

Fig.